# EUROPEAN PATENT APPLICATION

(11) **EP 0 693 481 A1**
(43) Date of publication of application: **24.01.1996**
(21) Application number: 95500085.6
(22) Date of filing: 14.06.1995
(51) Int. Cl.: C07D 233/72, A61K 31/415, A61K 31/495

(54) **Prodrugs of 5,5-diphenylhydantoin as antiepileptic and antiarrhythmic agents**

(30) Priority: 22.07.1994 ES 9401625
(71) Applicant: LABORATORIOS RUBIO, S.A., E-08022 Barcelona (ES)
(72) Inventor: Roca Estrem, Tomas, Laboratori de Quimica Organica, E-08028 Barcelona (ES); Bosch Cartes, Joan, Laborator. de Quimica Organica, E-08028 Barcelona (ES); Domenech Berrozpe, Josep, Departament de Farmacia, E-08028 Barcelona (ES); Obach Vidal, Rossend, Departement de Farmacia, E-08028 Barcelona (ES); Rubio Zurita, Pelayo, E-08022 Barcelona (ES)
(74) Representative: SUGRANES - VERDONCES - FERREGÜELA

(57) **Abstract**

The present invention deals with a product with the formula (I) which is a new prodrug of phenytoin, as well as its therapeutically acceptable solvates and addition salts. This product shows a solubility significantly higher than phenytoin itself. In addition, surprisingly, its hydrolisis rate is far higher than that of other known prodrugs of phenytoin, making it particularly useful as antiepileptic and antiarrhythmic agent.

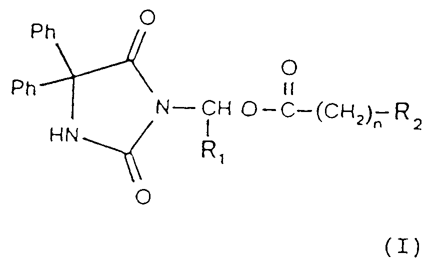

## Description

The present invention deals with new derivatives of 5,5-dyphenylhydantoine with the formula (I), their therapeutically acceptable solvates and addition salts, a procedure for its preparation and their use as phenytoin prodrugs (II).

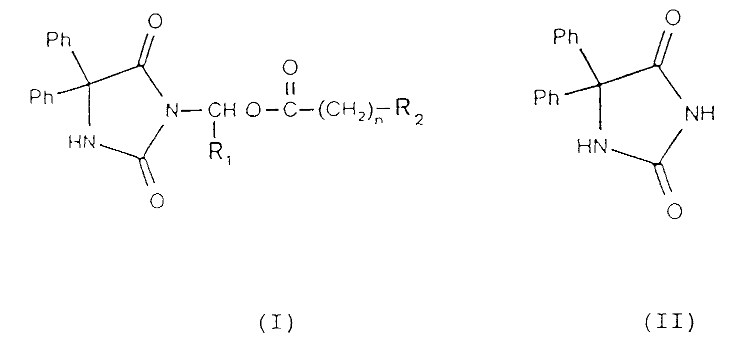

### STATE OF THE ART

There is currently a need for new compounds useful in the treatment of convulsive conditions and, particularly, epileptic seizures, as well as for new products with antiarrhythmic activity. The product with the formula (II), known as phenytoin (INN), as well as its sodium salt, is one of the compounds with these activities which has shown to be most useful. However, its low solubility in water makes its administration to patients difficult and seldom satisfactory. Phenytoin is given orally as the sodium salt in a strong alkaline solution, since it requires a pH between 10 an 12 to be maintained in solution. The alkalinity of this dosage form often causes gastric irritation which is a serious drawbacks for its use. Phenytoin can also be given by the intramuscular route, but the product commonly precipitates at the injection site, leading to unreliable blood levels of the drug. Moreover, absorption of intramuscular phenytoin is very slow, so it is not appropriate for treating epileptic seizures, in which a loading dose of the product is required. Intravenous administration of phenytoin is the most useful route in such cases. The most common formulation for intravenous administration contains 40% propylene glycol and 10% alcohol in water, with the solution adjusted to a high pH by sodium hydroxyde. However, this pharmaceutical composition has the effects associated with the risk of product precipitation and an excessively alkaline pH. An extensive review of the literature about intravenous administration of phenytoin appears in C.B. Tuttle, *Can. J. Hosp. Pharm.*, 1984, vol. 37, p. 137. The lack of adequate dosage forms for the administration of phenytoin emphasizes the need to create new compounds having adequate therapeutic properties to treat epilepsy and arrhythmia and devoid of the problems caused by the substances known. To date, efforts have been made to solve the problem by synthetizing new prodrugs of phenytoin which increase its solubility, described in patents US 4,163,058, US 4,260,769 and WO 9008128, amongst others. On the other hand, it is extremely important that, besides increasing the solubility of the active ingredient, the prodrug is rapidly hydrolized after injection, since the use of these drugs in a seizure requires high blood levels of the free active principle shortly after application. The attempts made to date to synthesize new prodrugs have been unsuccessful, since these are not hydrolized at a sufficient rate. Therefore, the synthesis of new prodrugs of phenytoin which increase its solubility and are rapidly hydrolized is a major problem in modern therapeutics.

### DESCRIPTION OF THE INVENTION

The present invention provides a family of products with the formula (I) 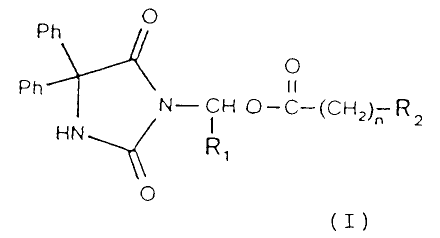
where n has a whole value from 1 to 5; R₁ represents H, phenyl or an alkyl radical C₁-C₄; and R₂ is selected from the radicals 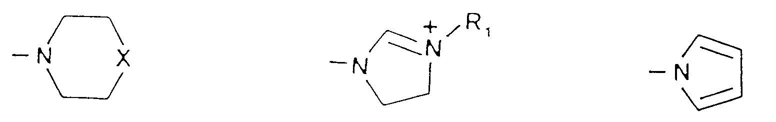
where -X- is -O- or 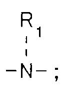
as well as its pharmaceutically acceptable solvates or addition salts, which act as phenytoin prodrugs. These products show a significantly higher solubility than phenytoin itself and its sodium salt, and are stable enought to be incorporated into conventional pharmaceutical formulations. Furthermore, their hydrolisis time is surprisingly low, once they have been given. The rapid hydrolisis of the prodrug is critical, since phenytoin is mainly used as an attack drug and a high blood level of the active principle is required as soon as possible.

The object of this invention is also a procedure for the preparation of compounds with the formula (I) which includes esterification of an acid with the formula (III) with an alcohol with the formula (IV) and, where appropriate, the addition of the relevant acid or base to form the salt desired.

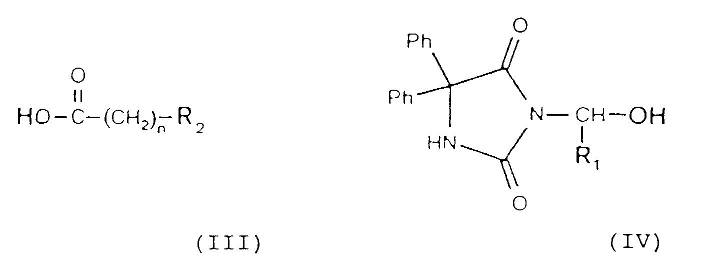

The attached diagram illustrates the procedure of preparation object of this invention. The diagram shows the substances described in the examples, where acid (VII) and alcohol (V) are particular cases of the products with general formulas (III) and (IV), respectively.

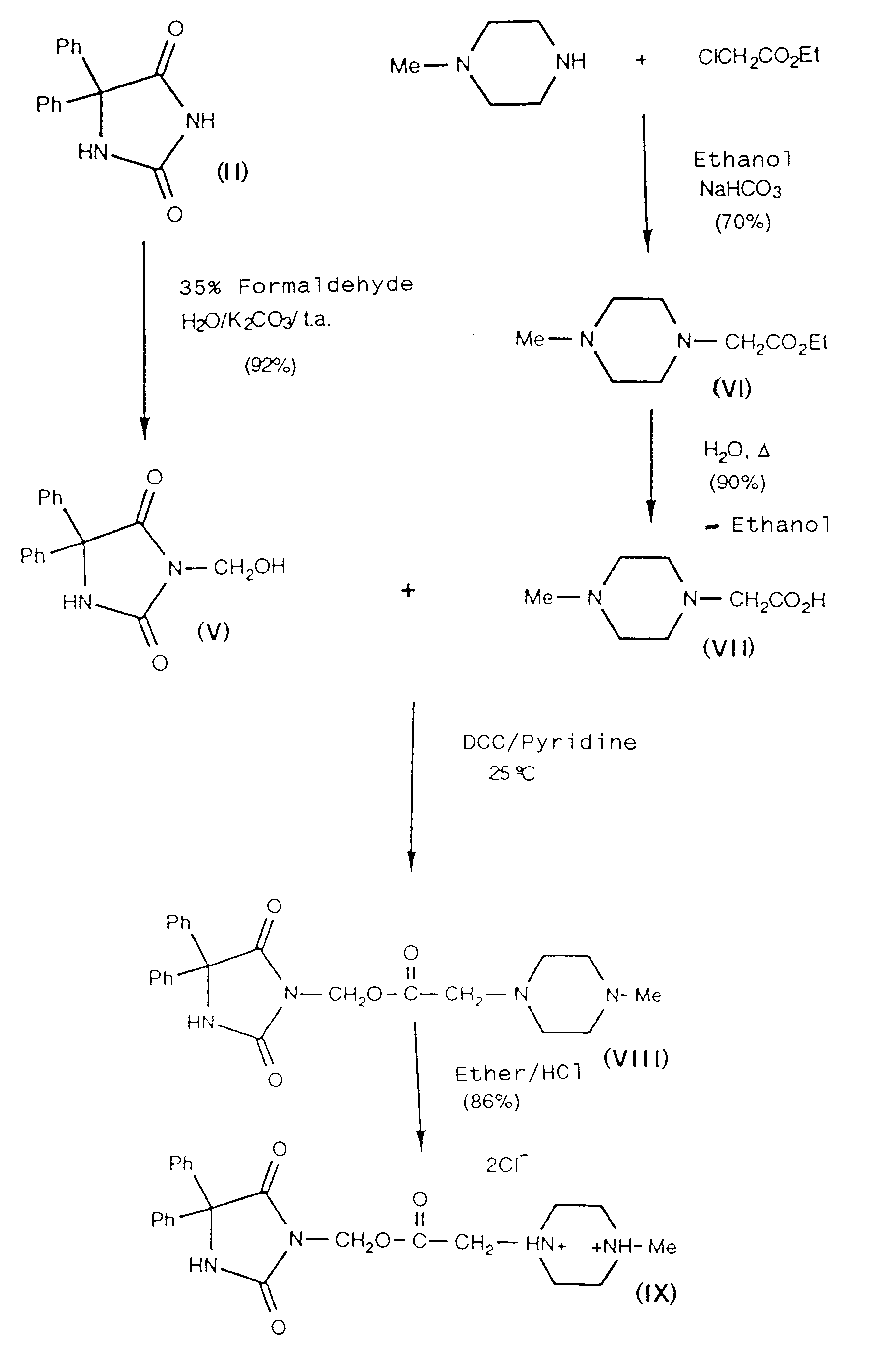

Obtention of starting products is also shown.

This invention also refers to pharmaceutical combinations containing a therapeutically effective amount of product (I), or a solvate or addition salt pharmaceutically acceptable, along with adequate amounts of pharmaceutically acceptable vehicles for intravenous administration.

As shown by the results given in Table 1 of Example 7, the hydrolisis of 5,5-diphenyl-3-[(4-methylpiperazinyl]-acetoxymethyl]-hydantoin dihydrochloride (IX) is virtually instantaneous. However, the data shown in Table 2 of Example 8 for blood hydrolisis of the disodium salt of the phosphoric ester of 5,5-diphenyl-3-(hydroxymethyl]hydantoin (XI), the prodrug currently on the market, show that the hydrolisis of this product is much more slower. The great difference between the hydrolisis rate of both compounds is amazing and, since these drugs are routinely used for treating seizures requiring a high blood level of the active principle to be reached quickly, the products object of this invention are potentially particularly useful and advantageous as anticonvulsant and antiarrhythmic agents.

### EXAMPLES

### Example 1: Preparation of 5,5-diphenyl-3-(hydroxymethyl) hydantoin (V)

A suspension containing 10 g (39.7 mmol) of phenytoin and 0.5 g (5 mmol) of potassium carbonate in 350 ml of water and 40 ml of 35% aqueous formaldehyde was stirred at room temperature for 24 h. By filtering the reaction mixture, then washing the solid with 25 ml of water and drying it in a vacuum oven at 50 °C in the presence of phosphorus pentoxide, 10.30 g (yield 92%) of 5,5-diphenyl-3-(hydroxymethyl) hydantoin (IV) with a melting point of 289-91 °C were obtained.

### Example 2: Preparation of (4-methylpiperazinyl) ethyl acetate (VI)

A suspension containing 20 g (199.6 mmol) of N-methylpiperazine, 21.20 ml (199.6 mmol) of ethyl chloroacetate and 20.08 g (239.5 mmol) of sodium bicarbonate in 250 ml ethanol was stirred at the reflux temperature of the solvent for 14 h. The reaction mixture was filtered, the solvent was removed at reduced pressure, and the residue obtained by vacuum distillation (80 °C; 12 mmHg) provided 26.04 g (yield 70%) of a colorless liquid identified as (4-methylpiperazinyl) ethyl acetate (VI).

### Example 3: Preparation of (4-methylpiperazinyl) acetic acid (VII)

A 22 g solution of (4-methylpiperaziny) ethyl acetate (VI) in 500 ml of water was stirred at the reflux temperature of the solvent for 2 h. The reaction mixture was evaporated to dryness, thus obtaining a residue which, by grinding with a 8:2 diethylether/acetone mixture, provided 16.75 g
(yield 90%) of a white solid with a melting point of 160-2 °C, identified as (4-methylpiperazinyl) acetic acid (VII).

### Example 4: Preparation of 5,5-diphenyl-3-[(4-methylpiperazinyl) acetoxymethyl]hydantoin (VIII)

### Method A:

A suspension containing 2.5 g (8.85 mmol) of 3-(hydroxymethyl)phenytoin (V), 1.65 g (8.85 mmol) of (4-methylpiperazinyl) acetic acid (VII) and 1.82 g (8.85 mmol) of dicyclohexylcarbodiimide in 20 ml of anhydrous pyridine was stirred, in a nitrogen atmosphere, at room temperature for 48 h. The resulting suspension was filtered, the solid was washed with 10 ml of dichloromethane and the filtration waters yielded after evaporation to dryness, a residue which was redissolved in dichloromethane (30 ml) and extracted with 20 ml of an aqueous solution of 8% hydrochloric acid. The aqueous phase was then alkalinized with sodium carbonate (pH = 9) and extracted with dichloromethane (3 x 20 ml). The organic phases, dried with anhydrous sodium sulfate and evaporated to dryness, provided a colorless syrup which was purified by column chromatography using alumina as adsorbent and a 15:4:1 mixture of hexane/dichloromethane/ethanol as eluent. By evaporating fractions 2 and 3, 0.76 g (yield 24%) of N,N'-dicyclohexyl-N-(4-methylpiperazinylacetyl) urea, melting point 118-20 °C, were obtained; its structure was confirmed by ¹H NMR and mass spectrometry. Fractions 7-11 provided, by evaporation and subsequent grinding with distilled hexane, 0.33 g (yield 9%) of 5,5-diphenyl-3-[4-methylpiperazinyl) acetoxymethyl]-hydantoin (VIII ) (m.p. 102-4 °C).

### Method B:

To a suspension of 5.57 g (35.40 mmol) of (4-methylpiperazinyl) acetic acid (VII) in 250 ml of distilled pyridine (previously dried at reflux with calcium oxide), cooled at 0-5 °C, 23.62 g (123.9 mmol) of p-toluenesulphonyl chloride were added under a nitrogen atmosphere. After 5 minutes, 10 g (35.40 mmol) of 5,5-diphenyl-3-(hydroxymethyl)hydantoin (V) were added, stirring the resulting solution for 2 h at the same temperature. The reaction mixture was poured over 300 ml of iced water, washed with diethylether (2 x 250 ml), and after alkalinization with a saturated solution of sodium carbonate, the aqueous phase was extracted three times with 350 ml of diethylether. The organic phases pooled, dried with anhydrous sodium sulphate, filtered and evaporated to dryness, provided a whitish foam which, after being redissolved with the minimum amount of dichloromethane and then precipitated and ground with 300 ml of distilled hexane, yielded 8.65 g (yield 58%) of a white solid with a melting point of 103-5 °C, identified as 5,5-diphenyl-3-[(4-methylpiperazinyl)acetoximethyl]hydantoin (VIII).
¹H NMR (DMSO-d₆;d, ppm): 2.11 (s, 3H); 2.23 (m, 4H); 2.41 (m, 4H); 3.19 (s, 2H); 5.48 (s, 2H); 7,38 (m, 10H); 9.92 (s, 1H).
¹³C NMR (DMSO-d₆;d, ppm): 46.0; 51.9; 54.8; 58.4; 61.7; 69.5; 126.9; 128.7; 128.9; 139.4; 153.7; 169.2; 172.7
IR (KBr, cm⁻¹): 1731 (CO), 1757 (CO) and 1790 (CO)
MS m/z (%) : 113 (73); 219 (62); 423 (M⁺+1, 100)
UV: lₘₐₓ 205 nm; log e= 4.467 lₘₐₓ 208 nm; log e = 4.457

### Example 5: Preparation of 5,5-diphenyl-3-[(4-methylpiperazinyl)-acetoxymethyl] hydantoin dihydrochloride (IX)

To a solution of 1.0 g (2.36 mmol) of 5,5-diphenyl-3-[(4-methylpiperazinyl)acetoxymethyl]hydantoin (VIII) in 25 ml acetone, a saturated solution of hydrochloric acid in diethylether was added. The precipitated white solid, filtered and washed with anhydrous acetone (10 ml), rendered 1.10 g (yield 86%) of 5,5-diphenyl-3-[(4-methylpiperazinyl)-acetoxymethyl]hydantoin dihydrochloride (IX) (M.P. = 206-8 °C). An aliquot was recrystallized with a 3:1:3 mixture of acetone/ethanol/ diethylether to obtain crystalls with a melting point at 208-10 °C which must be kept in the presence of a diseccating agent (anhydrous calcium chloride or silica gel).
¹H NMR (D₂O;d, ppm): 2.79 (s, 3H); 3.1-3.5 (ba, 8H); 3.73 (s, 2H); 5.50 (s, 2H); 7.24 (m, 10H)
¹³C NMR (D₂O d, ppm): 45.7; 52.1; 53.5; 58.4; 65.6; 73.3; 129.6; 131.9; 140.8; 157.8; 168.6; 176.5
IR (KBr, cm⁻¹): 1731 (CO) and 1780 (CO)
MS m/z (%): 113 (12); 421 (17); 423 (100)
AE Calc. for C₂₃Cl₂H₂₈O₄N₄:55.8% C; 5.7% H; 11.3% N
AE found:55.7% C; 5.7% H; 11.2% N

### Example 6: Preparation of 5,5-diphenyl-3-[(4-methylpiperazinyl)-acetoxymethyl]hydantoin disuccinate (X)

To a solution of 7.84 g (18.5 mmol) of 5,5-diphenyl-3-[(4-methylpiperazinyl)acetoxymethyl]hydantoin disuccinate (VIII) in 75 ml acetone, a solution of 4.60 g (39 mmol) succinic acid in 200 ml acetone was added. The mixture was stirred for 5 minutes at room temperature and concentrated at reduced pressure to a volume of 30 ml. Subsequently, 150 ml of diethylether were added and the gum resulting after grinding with the same solvent yielded 10.90 g (yield 90%) of a white solid identified as 5,5-diphenyl-3-[(4-methylpiperazinyl)-acetoxymethyl]hydantoin disuccinate (X), with a melting point of 76-8 °C, after drying in a vacuum oven at 35 °C for 3 days. The product must be kept in the presence of a diseccating agent (anhydrous calcium chloride or silica gel), since it is hygroscopic.
¹H NMR (DMSO-d₆; d, ppm): 2.26 (s, 3H); 2.40 (s, 8H); 2.49 (sa, 8H); 3.25 (s, 2H); 5,51 (s, 2H); 7,39 (m, 10H); 9.96 (s, 1H); 11.45 (ba, 2H)
¹³C NMR (DMSO-d₆; d, ppm): 29.6; 44.9; 51.0; 54.1; 58.0; 61.8; 65.5; 126.9; 128.7; 128.9; 139.4; 153.7; 169.2; 172.7; 174.3
IR (KBr, cm⁻¹): 1728 (CO) and 1780 (CO)
MS m/z (%): 113 (100); 159 (12); 423 (19)
UV:
1ₘₐₓ 203 nm; log e= 4.473
1ₘₐₓ 215 nm; log e= 4.174
Solubility (a= highly soluble; e= highly insoluble):
- water: a; methanol: a; DMSO: a; acetone: b; ether: d; hexane: e

### Example 7: Measurement of the hydrolisis rate of 5,5-diphenyl-3-[4-methylpiperazinyl)-acetoxymethyl]hydantoin dihydrochloride (IX) in whole rat blood

To study the hydrolisis of prodrugs in blood, Franz-type permeation cells of the company Crown Glass Co. Somerville, N.Y. have been used. The receiving compartment is used which allows for water recirculation using a glass packet in order to keep the system at 37 °C. This compartment is equipped with magnetic stirring.

For the experiment, male Wistar albino rats weighing 200-275 g, bred and selected at the stabularium of the School of Pharmacy of Barcelona, were used. The animals were fasted for 20 hours before starting the trials, but they were given water ad *libitum* throughout this period. In order to avoid coprophagy, the animals were placed in cages with a broad wire netting bottom.

After anesthesizing the rat, blood was drawn by cardiac puncture. A known amount of blood (10 ml) containing a prodrug concentration of 40 µg/ml was introduced through the lateral tubes of the receiving compartment of Franz cells. The system was kept at 37 °C and stirred magnetically, samples were taken (1 ml) and phenytoin was measured in each of them by HPLC.

The analytical methods were validated prior to estimating phenytoin levels in blood samples. The results obtained demonstrated that the analytical procedure used to test the hydrolisis of phenytoin prodrugs is accurate and precise, with a relative error ranging from -7.24% to 5.12% and a coefficient of variation from 11.86% to 4.07%.

Table 1 below shows the hydrolisis percentages in whole blood for three replicates of 5,5-diphenyl-3-[(4-methylpiperazinyl)-acetoxymethyl]hydantoin dihydrochloride (IX).

**TABLE 1**

| **% HYDROLISIS OF (IX) IN WHOLE BLOOD** | | | | |
|---|---|---|---|---|
| **TIME (min)** | **1** | **2** | **3** | **m ± s.d.** |
| 0.17 | 99.00 | 108.83 | 93.06 | 100.29 ± 7.96 |
| 0.33 | 85.63 | 95.83 | 125.60 | 102.35 ± 20.76 |
| 0.50 | 92.66 | 96.55 | 117.29 | 102.16 ± 13.24 |
| 1.5 | 95.83 | 107.78 | 108.58 | 104.06 ± 7.14 |
| 5.0 | 105.34 | 117.26 | 105.17 | 109.25 ± 6,92 |
| 16.0 | - | 118.66 | 110.47 | 114.56 ± 5.79 |
| 48.00 | 97.23 | 99.36 | 104.82 | 100.47 ± 3.91 |
| 115.00 | | 99.68 | - | - |

### Example 8: Measurement of the hydrolisis rate of the disodium salt of 5,5-diphenyl-3-(hydroxymethyl]hydantoin phosphoric ester (XI) in whole rat blood

As in the previous case, three replicates of the tested product were used. Table 2 shows the percentages of hydrolisis in whole blood of the product obtained in the experiment.

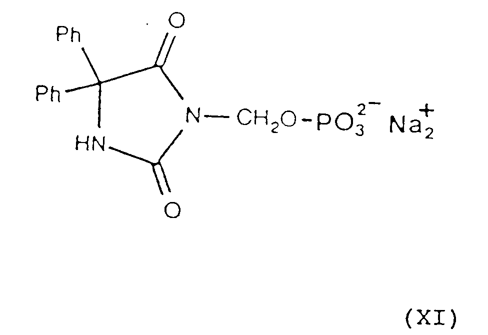

**TABLE 2**

| **% HYDROLISIS OF THE PRODUCT (XI) IN WHOLE BLOOD** | | | | |
|---|---|---|---|---|
| **TIME (min)** | **1** | **2** | **3** | **m ± s.d.** |
| 0.25 | 3.42 | 6.03 | - | 4.72 ± 1.84 |
| 0.50 | 6.37 | 7.41 | 11.65 | 8.47 ± 2.79 |
| 2.00 | 10.48 | 10.83 | 24.04 | 15.11 ± 7.72 |
| 7.00 | 21.44 | 32.04 | 46.00 | 33.16 ± 12.31 |
| 15.00 | 38.89 | 46.07 | 93.78 | 59.58 ± 29.83 |
| 60.00 | 74.47 | 80.98 | - | 77.72 ± 4.60 |
| 147.00 | 80.98 | 81.65 | - | 81.31 ± 0.47 |
| 189.00 | 79.27 | 97.73 | - | 88.50 ± 13.05 |

## Claims

1. Product characterized by the following formula: 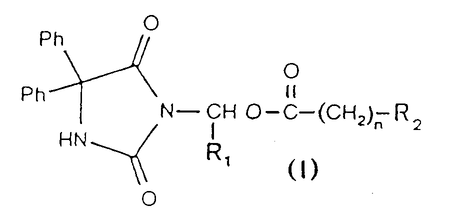 or a pharmaceutically acceptable solvate or addition salt thereof ; where n has a whole value from 1 to 5; R₁ represents H, phenyl or an alkyl radical C₁-C₄; and R₂ represents a radical selected from the following: 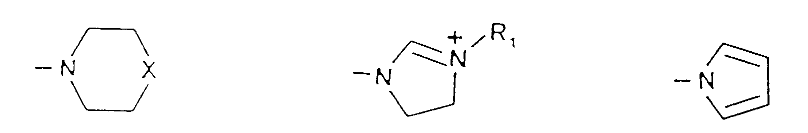 where -X- is -O- or

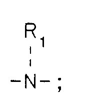

2. A product in accordance with claim 1 characterized by the name 5,5-diphenyl-3-[(4-methylpiperazinyl)-acetoxymethyl]hydantoin, with the formula:

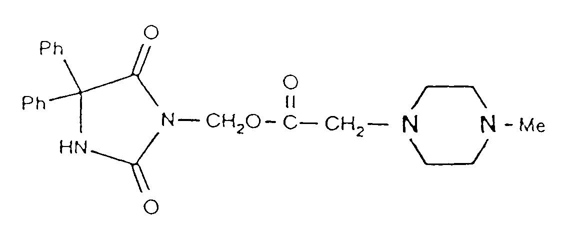

3. A product in accordance with any of the previous claims characterized by the addition salt being disuccinate.

4. A product complying with any of claims 1 and 2 characterized by the addition salt being dihydrochloride.

5. A pharmaceutical composition characterized by including a therapeutically effective amount of a product of any of the above claims along with adequate amounts of excipients for intravenous administration.

6. Use of a product of any of claims 1-4 for the preparation of an anticonvulsant drug.

7. Use of a product of any of claims 1-4 for the preparation of an antiarrhythmic drug.

8. A procedure for preparing the product described in claims 1-4 including the esterification of an acid with the formula (III) with an alcohol with the formula (IV) and, if applicable, the addition of the relevant acid or base to form the desired salt.
